# EUROPÄISCHE PATENTSCHRIFT

(11) **EP 2 341 881 B1**
(45) Veröffentlichungstag und Bekanntmachung des Hinweises auf die Patenterteilung: **18.03.2015**
(21) Anmeldenummer: 09749072.6
(22) Anmeldetag: 02.11.2009
(51) Int. Cl.: A61L 15/20, A61F 13/15, A61L 9/00, A61L 15/22, C08J 3/20, C08K 11/00, A61L 15/46, A61L 15/60, C08J 3/24, A61F 13/53

(54) **VERFAHREN ZUR HERSTELLUNG WASSERABSORBIERENDER POLYMERPARTIKEL**
METHOD FOR PRODUCING WATER-ABSORBING POLYMER PARTICLES
PROCÉDÉ DE FABRICATION DE PARTICULES POLYMÈRES ABSORBANT L'EAU

(30) Priorität: 07.11.2008 US 112454 P
(43) Veröffentlichungstag der Anmeldung: 13.07.2011
(73) Patentinhaber: BASF SE, 67056 Ludwigshafen (DE)
(72) Erfinder: BRAIG, Volker, 69469 Weinheim-Lützelsachsen (DE); DANIEL, Thomas, 67165 Waldsee (DE); JENTZSCH, Axel, 67063 Ludwigshafen (DE); BROCKMEYER, Andreas, 64665 Alsbach-Hähnlein (DE)
(86) Internationale Anmeldenummer: PCT/EP2009/064439
(87) Internationale Veröffentlichungsnummer: WO 2010/052182

(56) Entgegenhaltungen:
- EP-A1- 1 510 562
- WO-A2-2009/040106

## Beschreibung

Die vorliegende Erfindung betrifft ein Verfahren zur Herstellung geruchsinhibierender wasserabsorbierender Polymerpartikel auf Basis ethylenisch ungesättigter, säuregruppentragender Monomere, wobei die Säuregruppen zu 40 bis 70 mol-% neutralisiert sind und die Polymerpartikel mit einem kondensierten und/oder einem hydrolysierbaren Tannin beschichtet werden.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden zur Herstellung von Windeln, Tampons, Damenbinden und anderen Hygieneartikeln, aber auch als wasserzurückhaltende Mittel im landwirtschaftlichen Gartenbau verwendet.

EP 1 510 562 A1 beschreibt die Herstellung geruchsinhibierender wasserabsorbierender Polymerpartikel, wobei die Polymerpartikel mit Pflanzenextrakten, die u.a. Tannin oder Tanninsäure enthalten können, beschichtet werden.

WO 2006/109842 A1 beschreibt ein Verfahren zur Herstellung wasserabsorbierender Polymerpartikel, wobei eine Monomerlösung mit einem definierten Gehalt an Hydrochinonmonomethylether und Eisen und wenig Protoanemonin und Furfural polymerisiert wird.

Aufgabe der vorliegenden Erfindung war die Bereitstellung eines verbesserten Verfahrens zur Herstellung geruchsinhibierender wasserabsorbierender Polymerpartikel.

Gelöst wurde die Aufgabe durch ein Verfahren zur Herstellung wasserabsorbierender Polymerpartikel, umfassend
i) die Neutralisation mindestens eines ethylenisch ungesättigten, säuregruppentragenden Monomers mit einer Base bis zu einem Neutralisationsgrad von 40 bis 70 mol-%, wobei die Base weniger als 0,0005 Gew.-% Eisenionen enthält und die zur Förderung der Base verwendeten Leitungen aus Edelstahl oder einem polymeren Material sind,
ii) Polymerisation des neutralisierten Monomers in Gegenwart mindestens eines Vernetzers und mindestens eines Initiators, wobei der Initiator im Wesentlichen frei von Eisenionen ist,
iii) Trocknung des erhaltenen Polymergels,
iv) Zerkleinerung des getrockneten Polymergels zu Polymerpartikeln,
v) Klassierung der erhaltenen Polymerpartikel und
vi) optional Oberflächennachvernetzung der klassierten Polymerpartikel,
wobei die Polymerpartikel mit 0,01 bis 1 Gew.-% mindestens eines kondensierten und/oder eines hydrolysierbaren Tannins beschichtet werden.

Der Neutralisationsgrad beträgt vorzugsweise von 45 bis 65 mol-%, besonders bevorzugt von 48 bis 62 mol-%, ganz besonders bevorzugt von 50 bis 60 mol-%.

Die Base enthält vorzugsweise weniger als 0,0001 Gew.-%, besonders bevorzugt weniger als 0,00002 Gew.-%, ganz besonders bevorzugt weniger als 0,00001 Gew.-%, an Eisenionen.

Geeignete Edelstähle sind austenitische Stähle mit beispielsweise mindestens 0,08 Gew.-% Kohlenstoff. Vorteilhaft enthalten die austenitischen Stähle neben Eisen, Kohlenstoff, Chrom, Nickel und optional Molybdän noch weitere Legierungsbestandteile, vorzugsweise Niob oder Titan.

Die bevorzugten Edelstähle sind Edelstähle mit der Werkstoffnummer 1.45xx gemäß der DIN EN 10020, wobei xx eine natürliche Zahl zwischen 0 und 99 sein kann. Besonders bevorzugte Werkstoffe sind die Stähle mit den Werkstoffnummern 1.4541 und 1.4571, insbesondere Stahl mit der Werkstoffnummer 1.4541.

Geeignete polymere Materialien sind Polyethylen, Polypropylen, Polyester, Polyamid, Polytetrafluorethylen, Polyvinylchlorid, Epoxidharze und Silikonharze. Ganz besonders bevorzugt ist Polypropylen.

Die verwendeten Initiatorsysteme sind im Wesentlichen frei von Eisenionen, wobei die verwendeten Initiatorsysteme vorzugsweise weniger als 0,1 Gew.-%, besonders bevorzugt weniger als 0,01 Gew.-%, ganz besonders bevorzugt weniger als 0,001 Gew.-%, Eisenionen enthalten, jeweils bezogen auf die Gesamtmenge des Initiatorsystems.

Geeignete kondensierte Tannine sind polymere Flavan-3-ole auf Basis von Epikatechin und Katechin oder polymere Flavan-3,4-diole auf Basis von Leukopelargonidin.

Geeignete hydrolysierbare Tannine sind Ester aus Sacchariden, wie Glukose, mit Gallussäuren, wie Gallussäure, Galloylgallussäure und Digalloylgallussäure.

Die Polymerpartikel werden vorzugsweise mit 0,02 bis 0,5 Gew.-%, besonders bevorzugt 0,05 bis 0,3 Gew.-%, ganz besonders bevorzugt 0,1 bis 0,2 Gew.-%, kondensierten und/oder hydrolysierbaren Tanninen beschichtet.

Die Beschichtung der Polymerpartikel wird vorzugsweise mittels Mischern mit bewegten Mischwerkzeugen durchgeführt. Die zur Oberflächennachvernetzung einsetzbaren Mischer können auch für die erfindungsgemäße Beschichtung verwendet werden.

Es ist möglich die nach der Klassierung v) oder die nach der optionalen Oberflächennachvernetzung vi) erhaltenen Polymerpartikel mit einem Komplexbildner zu beschichten. Weiterhin ist es möglich die Beschichtung gleichzeitig mit der Oberflächennachvernetzung durchzuführen.

Vorzugsweise werden die Chelatbildner zur Beschichtung auf die Polymerpartikel als Lösung in einem geeigneten Lösungsmittel, vorzugsweise Wasser, aufgesprüht.

In einer bevorzugten Ausführungsform der vorliegenden Erfindung werden die wasserabsorbierenden Polymerpartikel mit einem Reduktionsmittel und/oder einem Zinksalz beschichtet.

Geeignete Reduktionsmittel sind beispielsweise Natriumsulfit, Natriumhydrogensulfit (Natriumbisulfit), Natriumdithionit, Sulfinsäuren und deren Salze, Ascorbinsäure, Natriumhypophosphit, Natriumphosphit, sowie Phosphinsäuren und deren Salze. Vorzugsweise werden aber Salze der unterphosphorigen Säure, beispielsweise Natriumhypophosphit, und Salze von Sulfinsäuren verwendet, beispielsweise das Dinatriumsalz der 2-Hydroxy-2-sulfinatoessigsäure. Als Reduktionsmittel wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite^{®} FF6 und Brüggotite^{®} FF7 (Brüggemann Chemicals; Heilbronn; DE) erhältlich.

Die eingesetzte Menge an Reduktionsmittel beträgt vorzugsweise 0,01 bis 5 Gew.-%, besonders bevorzugt 0,05 bis 2 Gew.-%, ganz besonders bevorzugt 0,1 bis 1 Gew.-%, jeweils bezogen auf das wasserabsorbierende Polymer.

Geeignete Zinksalze sind beispielsweise Zinkhydroxid, Zinksulfat, Zinkchlorid, Zinkcitrat, Zinkacetat und Zinklaktat. Vorzugsweise werden Zinksalze von Fettsäuren beispielsweise der Ricinolsäure eingesetzt.

Die eingesetzte Menge an Zinksalz beträgt vorzugsweise 0,01 bis 5 Gew.-%, besonders bevorzugt 0,05 bis 3 Gew.-%, ganz besonders bevorzugt 0,1 bis 1 Gew.-%, jeweils bezogen auf das wasserabsorbierende Polymer.

Die Reduktionsmittel bzw. Zinksalze werden üblicherweise als Lösung in einem geeigneten Lösungsmittel, vorzugsweise Wasser, eingesetzt.

Der vorliegenden Erfindung liegt die Erkenntnis zugrunde, dass die Kombination von leicht sauren wasserabsorbierenden Polymerpartikeln mit kondensierten und/oder hydrolysierbaren Tanninen zu einer deutlich verbesserten Geruchsinhibierung führt, gleichzeitig aber unerwünschte Verfärbungen auftreten. Die Verfärbungen werden durch Spuren an Eisenionen ausgelöst. Durch strenge Kontrolle der Menge an Eisenionen können nun wasserabsorbierende Polymerpartikel bereitgestellt werden, die einerseits eine gute Geruchsinhibierung und andererseits eine geringe Verfärbungsneigung aufweisen.

Als Quelle für Eisenionen kommt beispielsweise die häufig als Base verwendete Natronlauge in Betracht. Zur Durchführung des erfindungsgemäßen Verfahrens ist darauf zu achten, dass die verwendete Natronlauge einen möglicht geringen Anteil an Eisenionen aufweist.

Weiterhin sind die Rohrleitungen, in denen die Base der Neutralisation zugeführt wird, kritisch. So gilt Natronlauge gegenüber unlegierten Stählen nicht als korrosiv und wird sogar zur Passivierung verwendet. Allerdings löst Natronlauge aus unlegierten Stählen geringe Spuren an Eisenionen. Daher muss die Base mittels einer Rohrleitung aus Edelstahl oder einem polymeren Material in die Neutralisation gefördert werden. Aufgrund des damit verbundenen geringeren Eintrags an Eisenionen ist es vorteilhaft auch für die übrigen produktberührten Anlagenteile des Herstellverfahrens Edelstahl oder ein polymeres Material als Werkstoff zu verwenden.

Weiterhin ist darauf zu achten, dass ein Initiatorsystem verwendet wird, bei dem möglichst keine Eisenionen als Katalysator verwendet werden.

Durch zusätzliche Beschichtung der Polymerpartikel mit Reduktionsmitteln und/oder Zinksalzen kann die Verfärbungsneigung weiter günstig beeinflusst werden.

Im Folgenden wird die Herstellung der wasserabsorbierenden Polymerpartikel näher erläutert.

Die wasserabsorbierenden Polymerpartikel werden beispielsweise durch Polymerisation einer Monomerlösung oder -suspension, enthaltend
a) mindestens ein ethylenisch ungesättigtes, säuregruppentragendes Monomer, wobei die Säuregruppen zu 40 bis 70 mol-% neutralisiert sind,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure. Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite^{®} FF6 und Brüggolite^{®} FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 45 bis 65 mol-%, besonders bevorzugt von 48 bis 62 mol-%, ganz besonders bevorzugt von 50 bis 60 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Kaliumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA (European Disposables and Nonwovens Association) empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA (European Disposables and Nonwovens Association) empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C₁ Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben.

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar^{®} Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix^{®} (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex^{®} Horizontal Paddle Dryer (Hosokawa Micron GmbH; Leingarten; DE), Hosokawa Bepex^{®} Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verpackungsneigung der Polymerpartikel sind beispielsweise pyrogene Kieselsäure, wie Aerosil^{®} 200, und Tenside, wie Span^{®} 20.

Ein weiterer Gegenstand der vorliegenden Erfindung sind die gemäß dem erfindungsgemäßen Verfahren hergestellten wasserabsorbierenden Polymerpartikel.

Die gemäß dem erfindungsgemäßen Verfahren hergestellten wasserabsorbierenden Polymerpartikel weisen einen niedrigen Gehalt an Eisenionen auf. Der Gehalt an Eisenionen beträgt weniger als 0,001 Gew.-%, vorzugsweise weniger als 0,0005 Gew.-%, besonders bevorzugt weniger als 0,0001 Gew.-%, ganz besonders bevorzugt weniger als 0,00002 Gew.-%.

Wichtig ist auch ein ausreichender Überschuss an kondensierten und/oder hydrolysierbaren Tannin gegenüber den Eisenionen. Das Gewichtsverhältnis von Eisenionen zu kondensierten und/oder hydrolysierbaren Tannin beträgt weniger als 0,02, vorzugsweise weniger als 0,01, besonders bevorzugt weniger als 0,005, ganz besonders bevorzugt weniger als 0,001.

Die gemäß dem erfindungsgemäßen Verfahren hergestellten wasserabsorbierenden Polymerpartikel weisen einen Feuchtegehalt von vorzugsweise 1 bis 10 Gew.-%, besonders bevorzugt 2 bis 8 Gew.-%, ganz besonders bevorzugt 3 bis 5 Gew.-%, auf, wobei der Wassergehalt gemäß der von der EDANA (European Disposables and Nonwovens Association) empfohlenen Testmethode Nr. WSP 230.2-05 "Moisture Content" bestimmt wird.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA (European Disposables and Nonwovens Association) empfohlenen Testmethode Nr. WSP 241.2-05 "Centrifuge Retention Capacity" bestimmt.

Die gemäß dem erfindungsgemäßen Verfahren hergestellten wasserabsorbierenden Polymerpartikel weisen eine Absorption unter einem Druck von 49,2 g/cm² von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm² der wasserabsorbierenden Polymerpartikel beträgt üblicherweise weniger als 35 g/g. Die Absorption unter einem Druck von 49,2 g/cm² wird analog der von der EDANA (European Disposables and Nonwovens Association) empfohlenen Testmethode Nr. WSP 242.2-05 "Absorption under Pressure" bestimmt, wobei statt eines Drucks von 21,0 g/cm² ein Druck von 49,2 g/cm² eingestellt wird.

Ein weiterer Gegenstand der vorliegenden Erfindung sind Hygieneartikel, enthaltend gemäß dem erfindungsgemäßen Verfahren hergestellte wasserabsorbierende Polymerpartikel, insbesondere Hygieneartikel zur Damenhygiene, Hygieneartikel für leichte und schwere Inkontinenz oder Kleintierstreu.

### Beispiele

Folgende Lösungen wurden hergestellt:
1. 16,67 g Gallotannin (tannic acid; CAS no. [1401-55-4]; ABCR GmbH & Co. KG; DE) wurden in ein Weithalsglasgefäß eingewogen und mit dest. Wasser auf 100,02 g aufgefüllt. Zur vollständigen Lösung wurde das Gefäß in einem Wasserbad (ca. 40°C) leicht erwärmt.
2. 1,67 g Eisensulfat Hydrat (86,0-89.0% FeSO₄; CAS no. [13463-43-9]; Riedel-de Häen) wurden in ein Weithalsglasgefäß eingewogen und mit dest. Wasser auf 100,10 g aufgefüllt.
3. 16,72g Gallotannin (tannic acid; CAS no. [1401-55-4]; ABCR GmbH & Co. KG; DE) und 1,70g Eisensulfat Hydrat (86,0-89,0% FeSO₄; CAS no. [13463-43-9]; Riedel-de Häen) wurden in ein Weithalsglasgefäß eingewogen und mit dest. Wasser auf 100,13 g aufgefüllt. Zur vollständigen Lösung wurde das Gefäß in einem Wasserbad (ca. 40°C) leicht erwärmt.

20,0 g wasserabsorbierende Polymerpartikel (HySorb® B7065; BASF SE; DE) wurden in eine modifizierte Kaffeemühle (Blender 8012 Model 34BL99; Waring Laboratory; US) mit einem Aufsatz aus Edelstahl (Innendurchmesser 8 cm, Innenhöhe 4 cm, Werkzeugdurchmesser 7 cm, Zugabepunkt im Deckel 1,3 cm vom Rand entfernt, Stromstörer im Deckel) eingefüllt. Die modifizierte Kaffeemühle wurde auf Stufe 3 betrieben. Über eine Spritze mit Kanüle wurden jeweils 0,60 g der jeweiligen Lösung langsam zugegeben. Nach Ende der Zugabe wurden die wasserabsorbierenden Polymerpartikel in eine Glasschale umgefüllt und 30 Minuten bei 50°C im Trockenschrank getrocknet. Zur besseren Homogenisierung wurden die wasserabsorbierenden Polymerpartikel für 10 Minuten in einer Porzellanreibschale (Innendurchmesser 8,5 cm) mit Pistill verrieben.

Die Farbe der Proben wurde mit einem Spektrophotometer (LabScan XE; Hunter Associates Laboratory, Inc.; US) vermessen. Die wasserabsorbierenden Polymerpartikel (SAP) wurden in den Deckel einer Polystyrol-Petrischale (Innendurchmesser 3,9 cm) gefüllt und mit folgenden Einstellungen vermessen:
Potsize: 1,2 Zoll
Viewing Area: 1 Zoll
Illuminant: C
Observer: 2°

| SAP mit | L | a | b |
|---|---|---|---|
| Lösung 1 | 89,02 | -0,4 | 6,5 |
| Lösung 2 | 88,88 | -0,43 | 3,51 |
| Lösung 3 | 64,49 | 3,92 | 1,29 |

Man erkennt, dass die mit Lösung 3 behandelten wasserabsorbierenden Polymerpartikel einen kleineren L-Wert aufweisen und damit weniger weiß ist.

## Patentansprüche

1. Verfahren zur Herstellung wasserabsorbierender Polymerpartikel, umfassend
i) die Neutralisation mindestens eines ethylenisch ungesättigten, säuregruppentragenden Monomers mit einer Base bis zu einem Neutralisationsgrad von 40 bis 70 mol-%, wobei die Base weniger als 0,0005 Gew.-% Eisenionen enthält und die zur Förderung der Base verwendeten Leitungen aus Edelstahl und/oder einem polymeren Material sind,
ii) Polymerisation des neutralisierten Monomers in Gegenwart mindestens eines Vernetzers und mindestens eines Initiators, wobei der Initiator im Wesentlichen frei von Eisenionen ist,
iii) Trocknung des erhaltenen Polymergels,
iv) Zerkleinerung des getrockneten Polymergels zu Polymerpartikeln,
v) Klassierung der erhaltenen Polymerpartikel und
vi) optional Oberflächennachvernetzung der klassierten Polymerpartikel,
wobei die Polymerpartikel mit 0,01 bis 1 Gew.-% mindestens eines kondensierten und/oder eines hydrolysierbaren Tannins beschichtet werden.

2. Verfahren gemäß Anspruch 1, **dadurch gekennzeichnet, dass** die Polymerpartikel mit Aluminiumsulfat oder Aluminiumlaktat beschichtet werden.

3. Verfahren gemäß Anspruch 1 oder 2, **dadurch gekennzeichnet, dass** die Polymerpartikel mit mindestens einer Verbindung, die mit mindestens zwei Carboxylatgruppen der wasserabsorbierenden Polymerpartikel kovalente Bindungen bilden kann, oberflächennachvernetzt werden.

4. Verfahren gemäß Anspruch 3, **dadurch gekennzeichnet, dass** die oberflächennachvemetzten Polymerpartikel mit einer wässrigen Lösung nachbefeuchtet werden.

5. Verfahren gemäß einem der Ansprüche 1 bis 4, **dadurch gekennzeichnet, dass** die Polymerpartikel mit mindestens einem Reduktionsmittel beschichtet werden.

6. Verfahren gemäß einem der Ansprüche 1 bis 5, **dadurch gekennzeichnet, dass** die Polymerpartikel mit mindestens einem Zinksalz beschichtet werden.

7. Wasserabsorbierende Polymerpartikel, erhältlich durch Polymerisation einer wässrigen Monomerlösung oder -suspension, enthaltend
a) mindestens ein ethylenisch ungesättigtes, säuregruppentragendes Monomer, wobei die Säuregruppen zu 40 bis 70 mol-% neutralisiert sind,
b) mindestens einen Vernetzer und
c) mindestens einen Initiator,
wobei die Polymerpartikel weniger als 0,001 Gew.-% Eisenionen enthalten und mit 0,01 bis 1 Gew.-% mindestens eines kondensierten und/oder eines hydrolysierbaren Tannins beschichtet sind, wobei das Gewichtsverhältnis von Eisenionen zu kondensierten und/oder hydrolysierbaren Tannin weniger als 0,02 beträgt.

8. Polymerpartikel gemäß Anspruch 7, **dadurch gekennzeichnet, dass** die Polymer-partikel mit Aluminiumsulfat oder Aluminiumlaktat beschichtet wurden.

9. Polymerpartikel gemäß Anspruch 7 oder 8, **dadurch gekennzeichnet, dass** die Polymerpartikel mit einer Verbindung, die mit mindestens zwei Carboxylatgruppen der wasserabsorbierenden Polymerpartikel kovalente Bindungen bilden kann, oberflächennachvernetzt wurden.

10. Polymerpartikel gemäß einem der Ansprüche 7 bis 9, **dadurch gekennzeichnet, dass** mindestens 95% der Polymerpartikel eine Partikelgröße von 150 bis 600 µm aufweisen.

11. Polymerpartikel gemäß einem der Ansprüche 7 bis 10, **dadurch gekennzeichnet, dass** die Polymerpartikel einen Feuchtegehalt von 1 bis 10 Gew.% aufweisen.

12. Polymerpartikel gemäß einem der Ansprüche 7 bis 11, **dadurch gekennzeichnet, dass** die Polymerpartikel mit mindestens einem Reduktionsmittel beschichtet wurden.

13. Polymerpartikel gemäß einem der Ansprüche 7 bis 12, **dadurch gekennzeichnet, dass** die Polymerpartikel mit mindestens einem Zinksalz beschichtet wurden.

14. Polymerpartikel gemäß einem der Ansprüche 7 bis 13, **dadurch gekennzeichnet, dass** die Polymerpartikel eine Zentrifugenretentionskapazität von 15 bis 60 g/g aufweisen.

15. Polymerpartikel gemäß einem der Ansprüche 7 bis 14, **dadurch gekennzeichnet, dass** die Polymerpartikel eine Absorption unter einem Druck von 49,2 g/cm² von 15 bis 35 g/g aufweisen.

16. Hygieneartikel, enthaltend Polymerpartikel gemäß einem der Ansprüche 7 bis 15.

## Claims

1. A process for producing water-absorbing polymer particles, comprising
i) the neutralization of at least one ethylenically unsaturated monomer bearing acid groups with a base up to a degree of neutralization of from 40 to 70 mol%, the base comprising less than 0.0005% by weight of iron ions and the lines used to convey the base being made of stainless steel and/or a polymeric material,
ii) polymerization of the neutralized monomer in the presence of at least one crosslinker and of at least one initiator, the initiator being essentially free of iron ions,
iii) drying of the resulting polymer gel,
iv) comminution of the dried polymer gel to polymer particles,
v) classification of the resulting polymer particles and
vi) optional surface postcrosslinking of the classified polymer particles,
the polymer particles being coated with from 0.01 to 1% by weight of at least one condensed and/or a hydrolyzable tannin.

2. The process according to claim 1, wherein the polymer particles are coated with aluminum sulfate or aluminum lactate.

3. The process according to claim 1 or 2, wherein the polymer particles are surface postcrosslinked with at least one compound, which can form covalent bonds with at least two carboxylate groups of the water-absorbing polymer particles.

4. The process according to claim 3, wherein the surface postcrosslinked polymer particles are remoistened with an aqueous solution.

5. The process according to any one of claims 1 to 4, wherein the polymer particles are coated with at least one reducing agent.

6. The process according to any one of claims 1 to 5, wherein the polymer particles are coated with at least one zinc salt.

7. Water-absorbing polymer particles, obtainable by polymerizing an aqueous monomer solution or suspension, comprising
a) at least one ethylenically unsaturated monomer bearing acid groups wherein the acid groups have been neutralized to an extent of from 40 to 70 mol%,
b) at least one crosslinker and
c) at least one initiator,
the polymer particles comprising less than 0.001% by weight of iron ions and being coated with from 0.01 to 1% by weight of at least one condensed and/or hydrolyzable tannin, where the weight ratio of iron ions to condensed and/or hydrolyzable tannin is less than 0.02.

8. Polymer particles according to claim 7, which have been coated with aluminum sulfate or aluminum lactate.

9. Polymer particles according to claim 7 or 8, which have been surface postcrosslinked with a compound which can form covalent bonds with at least two carboxylate groups of the water-absorbing polymer particles.

10. Polymer particles according to any one of claims 7 to 9, at least 95% of which have a particle size of from 150 to 600 µm.

11. Polymer particles according to any one of claims 7 to 10, which have a moisture content of from 1 bis 10% by weight.

12. Polymer particles according to any one of claims 7 to 11, which have been coated with at least one reducing agent.

13. Polymer particles according to any one of claims 7 to 12, which have been coated with at least one zinc salt.

14. Polymer particles according to any one of claims 7 to 13, which have a centrifuge retention capacity of from 15 to 60 g/g.

15. Polymer particles according to any one of claims 7 to 14, which have an absorption under a pressure of 49.2 g/cm² of from 15 to 35 g/g.

16. A hygiene article comprising polymer particles according to any one of claims 7 to 15.

## Revendications

1. Procédé de fabrication de particules polymères absorbant l'eau, comprenant :
i) la neutralisation d'au moins un monomère éthyléniquement insaturé, portant des groupes acides, avec une base jusqu'à un degré de neutralisation de 40 à 70 % en moles, la base contenant moins de 0,0005 % en poids d'ions fer et les conduites utilisées pour le transport de la base étant en acier inoxydable et/ou en un matériau polymère, et
ii) la polymérisation du monomère neutralisé en présence d'au moins un agent de réticulation et d'au moins un initiateur, l'initiateur étant essentiellement exempt d'ions fer,
iii) le séchage du gel polymère obtenu,
iv) le broyage du gel polymère séché en particules polymères,
v) la classification des particules polymères obtenues et
vi) éventuellement la post-réticulation de surface des particules polymères classées,
les particules polymères étant revêtues avec 0,01 à 1 % en poids d'au moins un tanin condensé et/ou hydrolysable.

2. Procédé selon la revendication 1, **caractérisé en ce que** les particules polymères sont revêtues avec du sulfate d'aluminium ou du lactate d'aluminium.

3. Procédé selon la revendication 1 ou 2, **caractérisé en ce que** les particules polymères sont post-réticulées en surface avec au moins un composé pouvant former des liaisons covalentes avec au moins deux groupes carboxylate des particules polymères absorbant l'eau.

4. Procédé selon la revendication 3, **caractérisé en ce que** les particules polymères post-réticulées en surface sont post-humidifiées avec une solution aqueuse.

5. Procédé selon l'une quelconque des revendications 1 à 4, **caractérisé en ce que** les particules polymères sont revêtues avec au moins un réducteur.

6. Procédé selon l'une quelconque des revendications 1 à 5, **caractérisé en ce que** les particules polymères sont revêtues avec au moins un sel de zinc.

7. Particules polymères absorbant l'eau, pouvant être obtenues par polymérisation d'une solution ou suspension aqueuse de monomères, contenant :
a) au moins un monomère éthyléniquement insaturé, portant des groupes acides, les groupes acides étant neutralisés à hauteur de 40 à 70 % en moles,
b) au moins un agent de réticulation et
c) au moins un initiateur,
les particules polymères contenant moins de 0,001 % en poids d'ions fer et étant revêtues avec 0,01 à 1 % en poids d'au moins un tanin condensé et/ou hydrolysable, le rapport en poids entre les ions fer et le tanin condensé et/ou hydrolysable étant inférieur à 0,02.

8. Particules polymères selon la revendication 7, **caractérisées en ce que** les particules polymères sont revêtues avec du sulfate d'aluminium ou du lactate d'aluminium.

9. Particules polymères selon la revendication 7 ou 8, **caractérisées en ce que** les particules polymères sont post-réticulées en surface avec un composé pouvant former des liaisons covalentes avec au moins deux groupes carboxylate des particules polymères absorbant l'eau.

10. Particules polymères selon l'une quelconque des revendications 7 à 9, **caractérisées en ce qu'**au moins 95 % des particules polymères présentent une taille de particule de 150 à 600 µm.

11. Particules polymères selon l'une quelconque des revendications 7 à 10, **caractérisées en ce que** les particules polymères présentent une teneur en humidité de 1 à 10 % en poids.

12. Particules polymères selon l'une quelconque des revendications 7 à 11, **caractérisées en ce que** les particules polymères sont revêtues avec au moins un réducteur.

13. Particules polymères selon l'une quelconque des revendications 7 à 12, **caractérisées en ce que** les particules polymères sont revêtues avec au moins un sel de zinc.

14. Particules polymères selon l'une quelconque des revendications 7 à 13, **caractérisées en ce que** les particules polymères présentent une capacité de rétention centrifuge de 15 à 60 g/g.

15. Particules polymères selon l'une quelconque des revendications 7 à 14, **caractérisées en ce que** les particules polymères présentent une absorption sous une pression de 49,2 g/cm² de 15 à 35 g/g.

16. Article d'hygiène, contenant des particules polymères selon l'une quelconque des revendications 7 à 15.
